# EUROPEAN PATENT APPLICATION

(11) **EP 3 409 202 A1**
(43) Date of publication of application: **05.12.2018**
(21) Application number: 18173061.5
(22) Date of filing: 17.05.2018
(51) Int. Cl.: A61B 5/145

(54) **BLOOD SUGAR MEASURING APPARATUS**

(30) Priority: 02.06.2017 TW 106118336
(71) Applicant: Phoenix Silicon International Corp., Hsinchu 300 (TW)
(72) Inventor: YANG, MIIN-TSONG, 300 Hsinchu (TW); WU, PO-CHIH, 300 Hsinchu (TW); KAO, TZU-PAI, 300 Hsinchu (TW); LIAO, HSUEH-CHUAN, 300 Hsinchu (TW); CHEN, TZU-HUI, 300 Hsinchu (TW); CHEN, CHAO-JUNG, 300 Hsinchu (TW)
(74) Representative: Lermer, Christoph

(57) **Abstract**

A blood sugar measuring apparatus includes a carrier, a measuring unit installed in the carrier, a calculation unit installed in the carrier and electrically connected to the measuring unit, a display unit installed in the carrier and electrically connected to the calculation unit, and a power unit installed in the carrier and electrically connected to the measuring unit, the calculation unit and the display unit. The blood sugar measuring apparatus is easy to measure the blood sugar without a significant psychological burden and discomfort, but with higher accuracy.

## Description

### CROSS REFERENCE TO RELATED APPLICATION

This application claims the benefit of Taiwan Patent Application Serial No. 106118336, filed June 2nd, 2017, the subject matter of which is incorporated herein by reference.

### BACKGROUND OF THE INVENTION

### (1) Field of the Invention

The invention relates to a blood sugar measuring apparatus, and more particularly to the measuring apparatus that is easier to measure the blood sugar without a psychological burden and discomfort, but with higher accuracy.

### (2) Description of the Prior Art

Refined diet to people's daily life has made worst clinical healthy concerns in three hypers; i.e. high blood sugar (or said as blood glucose), high blood cholesterol and hypertension. In particular, the high blood sugar usually leads to a diabetes, from which various cardiovascular diseases, strokes, chronic kidney diseases, diabetic feet, retinopathy and so on, would then be induced to jeopardize people's lives.

Hence, frequent or daily measuring the blood sugar is particularly crucial to patients with diabetes or high blood sugar. In the art, measuring methods of blood sugar include an invasive type and a non-invansive type.

The invasive type of the blood sugar measuring method is firstly to generate a micro cut at a finger tip or a specific body portion of a person to be tested, then to collect a specific amount of blood from this micro cut, and finally to obtain a corresponding blood sugar level from analyzing the collected blood.

On the other hand, the non-invasive type of the blood sugar measuring method is firstly to project a light with a specific spectrum onto a skin of the person to be tested, and then to obtain a corresponding blood sugar level from evaluating the reflected light.

Nevertheless, since a micro cut is a must for carrying out the invasive type of the blood sugar measuring method, thus unnecessary psychological burden or physical response are inevitable to some of the person's to be tested. However, to the non-invasive type of the blood sugar measuring method, inaccuracy measurements are also possible due to inappropriate light refraction, projection angling and/or light transmittance. Thus, since specific shortcomings do occur at different types of the conventional blood sugar measuring apparatuses, so there is definitely a room for improving the existing blood sugar measuring apparatuses.

### SUMMARY OF THE INVENTION

Accordingly, it is an object of the present invention to provide a blood sugar measuring apparatus, that does not need a micro cut at the person to be tested, can make easier the measurement of the blood sugar, and can provide an accurate blood sugar level.

In the present invention, the blood sugar measuring apparatus includes:
a carrier;
a measuring unit, located at the carrier;
a calculation unit, located inside the carrier and electrically coupled with the measuring unit;
a display unit, located inside the carrier and electrically coupled with the calculation unit; and
a power unit, located at the carrier to electrically couple the measuring unit, the calculation unit and the display unit.

By providing the blood sugar measuring apparatus of the present invention, a human tissue such as a saliva, a body tissue or a sweat, can be used for detecting the blood sugar level. Thereupon, the psychological fear or discomfort risen from being measured by the conventional invasive-type blood sugar measuring apparatus can be avoided, and the measurement accuracy can be increased. Thus, the blood sugar measuring apparatus of the present invention can detect the blood sugar easier under a situation of no psychological burden, no discomfort, but having higher accuracy.

All these objects are achieved by the blood sugar measuring apparatus described below.

### BRIEF DESCRIPTION OF THE DRAWINGS

The present invention will now be specified with reference to its preferred embodiment illustrated in the drawings, in which:
FIG.1 is a schematic view of a first embodiment of the blood sugar measuring apparatus in accordance with the present invention;
FIG.2 is a schematic view of a second embodiment of the blood sugar measuring apparatus in accordance with the present invention; and
FIG.3 is a block view of FIG.2.

### DESCRIPTION OF THE PREFERRED EMBODIMENT

The invention disclosed herein is directed to a blood sugar measuring apparatus. In the following description, numerous details are set forth in order to provide a thorough understanding of the present invention. It will be appreciated by one skilled in the art that variations of these specific details are possible while still achieving the results of the present invention. In other instance, well-known components are not described in detail in order not to unnecessarily obscure the present invention.

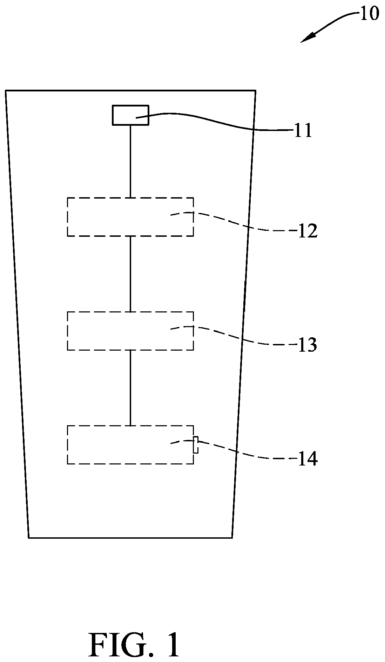

Referring now to FIG.1, a schematic view of a first embodiment of the blood sugar measuring apparatus in accordance with the present invention is shown. In this embodiment, the blood sugar measuring apparatus includes a carrier 10, a measuring unit 11, a calculation unit 12, a power unit 13 and a display unit 14.

The carrier 10 is configured to be a cup or a container. The measuring unit 11 is located at a rim of the carrier 10. The calculation unit 12 and the display unit 14 are both located inside the carrier 10. The power unit 13 can go located either inside or outside the carrier 10.

The measuring unit 11 is electrically coupled with the calculation unit 12. The calculation unit 12 is electrically coupled with the display unit 14. The measuring unit 11, the calculation unit 12 and the display unit 14 are all electrically coupled with the power unit 13.

In this embodiment, since the carrier 10 is embodied to be a cup orca container, thus it can be filled with a liquid such as water, tea, soft drink, coffee or any the like. As a use takes the liquid contained in the carrier 10, the lower lip of the user would contact the measuring unit 11 located at the rim of the carrier 10.

The lower lip can contain a human tissue, the saliva for example. In one example, if the measuring unit 11 is embodied as a loop for detecting the blood sugar, then an input of the measuring unit 11 can be a human tissue. The human tissue and a surface material on the measuring unit 11 can react to undergo an oxidation-reduction reaction, such that a voltage drop can be formed and then transmitted to the calculation unit 12. The calculation unit 12 bases on the voltage drop to calculate a corresponding blood sugar level in the user's body. The calculated blood sugar level is then transmitted to the display unit 14. The display unit 14 displays the blood sugar level to the user for him/her to understand the blood sugar level in his/her own body.

In another example, if the measuring unit 11 is embodied as an impedance-detecting apparatus having a detection electrode to be covered by the aforesaid human tissue. The impedance-detecting apparatus implements the detection electrode to obtain a corresponding impedance value, and then the impedance value is transmitted to the calculation unit 12 for the calculation unit 12 to base on the impedance value to calculate the blood sugar level inside the human body. The calculated blood sugar level is then transmitted to the display unit 14 for the display unit 14 to display the blood sugar level. Thereupon, the user can understand the blood sugar level inside his/her body.

In a further example, the measuring unit 11 is embodied to have a sensor and a loop for detecting the blood sugar. The sensor is to detect the body pressure of the user so as to proceed voltage feedback and further to start the loop for detecting blood sugar. In this example, an input of the loop for detecting the blood sugar can be a human tissue. The human tissue and the surface material of the loop for detecting blood sugar are involved in an oxidation-reduction reaction so as to form a voltage drop to be transmitted to the calculation unit 12. The calculation unit 12 bases on the voltage drop top calculate the blood sugar level in user's body. The blood sugar level is then transmitted to the display unit 14. The display unit 14 displays the blood sugar level for the user to understand his/her blood sugar level.

In the aforesaid oxidation-reduction reaction, the calculation unit 12 and the display unit 14 are energized by the power unit 13. In the present invention, the power unit 13 can be a general battery, an accumulator battery or a lithium battery. The carrier 10 can further include a connection port (not shown in the figure). The connection port is electrically coupled with the power unit 13, and can be a USB (Universal serial bus). Through the connection port, the power unit 13 can be charged.

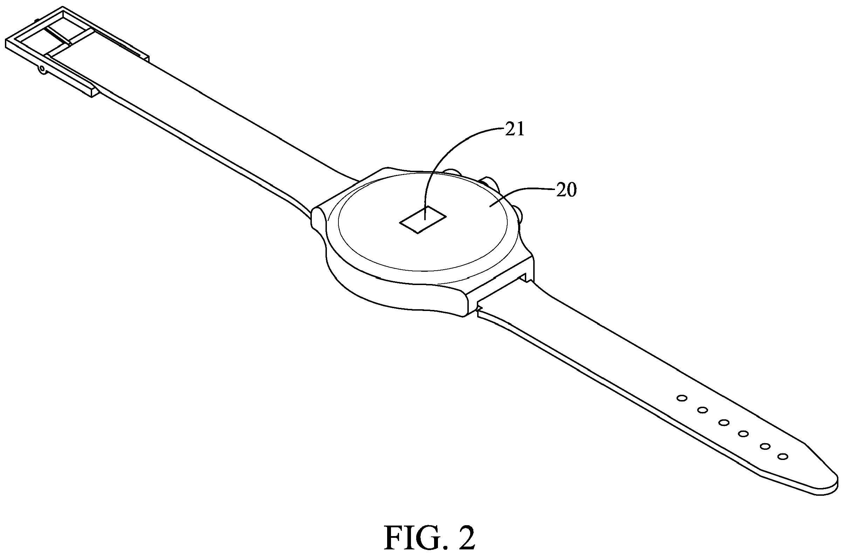

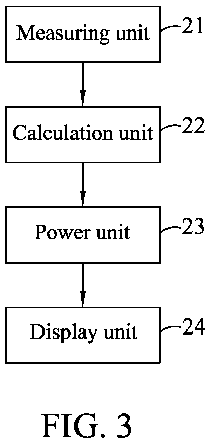

Referring now to FIG.2 and FIG.3, a schematic view and a block view of a second embodiment of the blood sugar measuring apparatus in accordance with the present invention are shown, respectively. In this embodiment, the blood sugar measuring apparatus includes a carrier 20, a measuring unit 21, a calculation unit 22, a power unit 23 and a display unit 24.

The carrier 20 can be a wearable device, such as a wristband, a necklace or a watch.

The measuring unit 21 is located at one surface of the carrier 20. The calculation unit 22 and the display unit 24 are located inside the carrier 20. The power unit 23 is located at the carrier 20.

The measuring unit 21 is electrically coupled with the calculation unit 22. The calculation unit 22 is electrically coupled with the display unit 24. The measuring unit 21, the calculation unit 22 and the display unit 24 are all electrically coupled with the power unit 23.

As described above, the carrier 20 is a wearable device, and thus can be worn at any appropriate portion of user's body, such as the hand or the neck.

While the present invention has been particularly shown and described with reference to a preferred embodiment, it will be understood by those skilled in the art that various changes in form and detail may be without departing from the spirit and scope of the present invention.

## Claims

1. A blood sugar measuring apparatus, comprising:
a carrier;
a measuring unit, located at the carrier;
a calculation unit, located inside the carrier, electrically coupled with the measuring unit;
a display unit, located inside the carrier, electrically coupled with the calculation unit; and
a power unit, located at the the carrier, electrically coupled with the measuring unit, the calculation unit and the display unit.

2. The blood sugar measuring apparatus of claim 1, wherein the carrier is one of a cup, a container and a wearable device.

3. The blood sugar measuring apparatus of claim 1, wherein the measuring unit is one of an impedance-detecting apparatus and a loop for detecting the blood sugar.

4. The blood sugar measuring apparatus of claim 1, wherein the power unit is one of a battery, an accumulator battery and a lithium battery.

5. The blood sugar measuring apparatus of claim 1, wherein the carrier has a connection port electrically coupled with the power unit.
